# EUROPEAN PATENT APPLICATION

(11) **EP 2 292 207 A1**
(43) Date of publication of application: **09.03.2011**
(21) Application number: 09754443.1
(22) Date of filing: 27.05.2009
(51) Int. Cl.: A61K 8/25, A61K 8/29, A61K 8/31, A61Q 1/00

(54) **COSMETIC MATERIAL AND COSMETIC METHOD FOR TOUCH UP**

(30) Priority: 29.05.2008 JP 2008141418
(71) Applicant: Shiseido Company, Ltd., Chuo-ku Tokyo 104-8010 (JP)
(72) Inventor: TOUYAMA, Keisuke, Yokohama-shi Kanagawa 224-8558 (JP); JOUICHI, Kyoko, Yokohama-shi Kanagawa 224-8558 (JP); KUSUMOTO, Takahiro, Yokohama-shi Kanagawa 224-8558 (JP)
(74) Representative: Henkel, Feiler & Hänzel
(86) International application number: PCT/JP2009/002345
(87) International publication number: WO 2009/144934

(57) **Abstract**

It is intended to provide a cosmetic composition suitable for touch-up that has both of such simplicity that anyone can easily use it, as with a face powder, and a natural, beautiful finish with a good coverage, as with a foundation, and to provide a cosmetic method using the same. The present invention provides a solid powdery cosmetic composition for touch-up comprising: a) 10 to 60% talc in a plate form having an average particle size D50 of 15 to 40 µm; b) 6 to 12% pigment-grade titanium oxide; and c) 6 to 9% oil, and a cosmetic method using the cosmetic composition. In the cosmetic composition of the present invention, the viscosity of the oil is preferably 100 cps or lower.

## Description

### Technical Field

The present invention relates to a cosmetic composition suitable particularly for touch-up. More specifically, the present invention relates to a cosmetic composition for touch-up formulated with talc in a plate form having a particular particle size, pigment-grade titanium oxide, and oil at a particular ratio, and a cosmetic touch-up method using the same.

### Background Art

For cosmetic compositions, particularly, makeup cosmetic compositions, the lasting of their effects over a long period, i.e., a long lasting is important. For example, Patent Document 1 discloses a solid powdery makeup cosmetic composition that has a duration improved by formulating therein highly viscous dimethylpolysiloxane, a high-aspect ratio powder muscovite or synthetic fluorphlogopite, and so on, and offers a smooth touch.

However, even makeup finished using such a cosmetic material having an improved duration inevitably comes off in such a way that it is smudged, slipped off, or faded over time due to sebum secretion, influence from the outside world, or the like.
When makeup comes off, so-called touch-up is required. Conventional touch-up has adopted, for example, a method involving wiping off a smudged foundation using a cleansing sheet and touching up the makeup, a method involving removing sebum using an oil blotting paper and putting on a face powder, or a method involving applying several layers of usual foundation.

However, the touch-up after makeup removal had the problem that it is very laborious. Moreover, when a face powder is put on after removal of sebum using an oil blotting paper or the like, the face powder itself has a poor coverage and hardly offers a satisfactory finish, due to its low cover-up, albeit with little so-called unevenness. On the other hand, when a usual foundation with a high cover-up is used, it has a good coverage but becomes a so-called caked-on state and tends to cause uneven application of makeup and have an unnatural finish. Hereinafter, these problems will be described briefly.

In general, conventional face powders are often applied onto a foundation and therefore adopt a low-coverage formula to prevent uneven application and make layers of face powder less conspicuous. Specifically, the amount of pigment-grade titanium oxide formulated in a face powder is often set to 5% or lower to control its coverage. Moreover, since a large amount of oil causes makeup to come off, the amount of the oil is also set to 5% or lower, in general. Although face powders having such characteristics have the advantage that they are easily layered and easily used in touch-up, these face powders tended to result in an insufficient coverage and a powdery finish.

On the other hand, foundations are cosmetic materials for which a coverage and a finish are important. Therefore, the amount of pigment-grade titanium oxide formulated therein is often set to 10% or higher. In addition, the amount of oil is also often set to around 10% or 10% or higher, because great importance is also placed on removability/applicability. Conventional general foundations are designed to give the best finish in first application, specifically, when applied after skincare or, in some cases, after application of a sun screen or a makeup base. Therefore, the foundations had a beautiful finish immediately after first application but tended to be applied conspicuously unevenly or thickly, albeit with a high coverage, when layered after coming off of makeup. Therefore, foundations were often unsatisfactory to general consumers as convenient touch-up means.

Thus, both conventional face powders and foundations have disadvantages that may become a problem particularly in use in touch-up. A cosmetic material that has the advantages of both of them and is suitable particularly for touch-up has not been obtained yet.
Patent Document 1: JP-A-Hei 9-249531

### Summary of the Invention

### Problem to be solved by the Invention

An object of the present invention is to solve the disadvantages of the conventional techniques described above. Specifically, an object of the present invention is to provide a cosmetic composition suitable particularly for touch-up that has both of such convenience that anyone can easily use it, as with a face powder, and a natural, beautiful finish with a good coverage, as with a foundation, and to provide a cosmetic method using the same.

### Means for Solving to Problem

To attain the object, the present invention provides a solid powdery cosmetic composition for touch-up comprising:
a) 10 to 60% by weight of talc in a plate form having an average particle size (D50) of 15 to 40 µm;
b) 6 to 12% by weight of pigment-grade titanium oxide; and
c) 6 to 9% by weight of oil, and a cosmetic method using the cosmetic composition.

### Effects of the Invention

A cosmetic composition and a cosmetic method of the present invention are suitable particularly for touch-up and can modify dullness attributed to coming off of makeup and reproduce a beautiful, natural finish of fresh makeup applied in the morning. Moreover, the cosmetic composition and the cosmetic method of the present invention can offer a beautiful finish with a luster and a moderate coverage, without providing a powdery finish.

### Modes for Carrying Out the Invention

A cosmetic composition of the present invention is a solid powdery cosmetic composition that contains talc in a plate form having the particular particle size, pigment-grade titanium oxide, and oil, which are formulated in particular amounts.
In general, talc used in cosmetics is powder obtained by pulverizing raw material minerals. The talc in a plate form constituting the present invention is high-aspect ratio talc powder particularly having an aspect ratio of 10 or higher, preferably 20 or higher, and is discriminated from low-aspect ratio talc in a block form. However, the talc in a plate form of the present invention may be a mixture of talc in a plate form and talc in a block form as long as its average aspect ratio is 10 or higher, preferably within the range of 10 to 100.

The talc in a plate form used in the present invention has its average particle size (D50) of 15 µm or larger, preferably within the range of 15 to 40 µm. When the average particle size (D50) is smaller than 15 µm, the resulting cosmetic composition tends to have a powdery finish and be inferior in luster.
Furthermore, for the talc in a plate form according to the present invention, it is preferred that powder having a small particle size should be removed therefrom by fractionation treatment. Use of talc in a plate form whose average particle size (D10) is set to preferably 4 µm or larger, more preferably 8 µm or larger, even more preferably 14 µm or larger, by fractionation treatment makes the spreadability of the cosmetic composition smoother, further reduces a powdery finish, and further improves a luster.

In the present invention, talc in a plate form conventionally used in cosmetic materials can be used as long as it satisfies the conditions described above. For example, the talc in a plate form used in the present invention may be produced by cracking raw material minerals by a usual method, preferably by a wet process, or a commercially available product may be used directly. Examples of preferable commercially available products can include Talclear LH series (manufactured by Nippon Talc Co., Ltd.), Silky Talc series (manufactured by YAMAGUCHI MICA CO., LTD.), and Fit Powder series (manufactured by YAMAGUCHI MICA CO., LTD.).

Moreover, the talc in a plate form of the present invention may be subjected to surface treatment performed on usual powder for cosmetics, for example, surface treatment with silicone, metallic soap, lecithin, amino acid, collagen, a fluorine compound, or the like, without impairing the effect thereof.

The amount of the talc in a plate form formulated in the cosmetic composition of the present invention is 10 to 60% by mass, preferably 30 to 60% by mass. When it is formulated in an amount exceeding 60% by mass, the resulting cosmetic composition tends to have poor removability/applicability, albeit with a satisfactory powdery finish or luster.

The pigment-grade titanium oxide used in the present invention needs only to be titanium oxide having a pigment-grade particle size conventionally used for imparting a cover-up (coverage) to cosmetic materials. The particle size of the pigment-grade titanium oxide is usually 0.2 to 0.4 µm in terms of an average primary particle size. The pigment-grade titanium oxide may or may not be subjected to surface treatment as described above for the talc in a plate form.

The amount of the pigment-grade titanium oxide formulated in the cosmetic composition of the present invention is 6 to 12% by mass, preferably 8 to 10% by mass. When it is formulated in an amount smaller than 6% by mass, the resulting cosmetic composition does not have a satisfactory coverage. When it is formulated in an amount exceeding 12% by mass, the resulting cosmetic composition may be cakey, albeit with a sufficient coverage.

Oil usually used in cosmetic materials can be used as the oil used in the cosmetic composition of the present invention. Examples of liquid oil include liquid paraffin, squalane, lanolin derivatives, higher alcohols, various ester oils, silicone oil, polyalkylene glycol polyether and other carboxylic acids, oligoester compounds, and terpene hydrocarbon oils. Examples of solid oil include ceresin wax, carnauba wax, polyethylene wax, paraffin wax, candelilla wax, microcrystalline wax, behenic acid, behenyl alcohol, Japan tallow, bees wax, and cetanol.

The amount of the oil formulated in the present invention is 6 to 10% by mass, preferably 6 to 8% by mass. This amount is characteristically comparable to the amount of oil in conventional face powders and set to an amount smaller than that in conventional foundations. When the amount of the oil is smaller than 6% by mass, the resulting cosmetic composition has a reduced fit and impact resistance. When the amount of the oil exceeds 10% by mass, the resulting cosmetic composition offers a heavy touch due to deteriorated removability/applicability and spreadability. In the present invention, the amount of the oil means the total weight of a mixture of liquid oil, solid oil, and organic surfactants such as sorbitan sesquiisostearate. Moreover, for example, oil-soluble UV absorbers such as octyl methoxycinnamate are also calculated as the oil.

Moreover, the oil formulated in the cosmetic composition of the present invention has a viscosity (25°C) of preferably 100 cps or lower, more preferably 30 cps or lower, in terms of the viscosity of the whole oil mixture. When the oil is formulated such that the viscosity of the mixture exceeds 100 cps, the resulting cosmetic composition tends to have deteriorated removability/applicability and spreadability, a powdery finish, and reduced luster.

Furthermore, it is preferred that the cosmetic composition of the present invention should be formulated with silicone elastomer powder. The formulation of this silicone elastomer powder further improves the removability/applicability and spreadability of the cosmetic composition and further reduces a powdery finish. The silicone elastomer powder formulated in the cosmetic composition of the present invention is not particularly limited and is generally powder, preferably in a spherical form, having a particle size on the order of a few µm to 30 µm.

A commercially available product can be used directly as such silicone elastomer powder. Examples of the commercially available product can include TORAYFIL E series manufactured by Dow Corning Toray Co., Ltd. and KSP series manufactured by Shin-Etsu Chemical Co., Ltd. Moreover, composite silicone elastomer powder having a surface coated with a clay mineral such as talc, sericite, kaolin, mica, or mica titanium may be used (see e.g., JP-A-2007-22951).
The silicone elastomer powder is formulated in an amount of preferably 2 to 6% by mass, more preferably 3 to 5% by mass, for use.

In the cosmetic composition of the present invention, other ingredients usually used in cosmetic compositions may be formulated arbitrarily without impairing the intended effect thereof. Examples of such ingredients include powders (except for the talc in a plate form, the pigment-grade titanium oxide, and the silicone elastomer powder described above). However, these ingredients are not limited to these examples.

Examples of the powders include: inorganic powders such as talc (except for the talc in a plate form described above), kaolin, sericite, muscovite, phlogopite, synthetic mica, lepidolite, biotite, lithium mica, vermiculite, magnesium carbonate, calcium carbonate, aluminum silicate, barium silicate, calcium silicate, magnesium silicate, strontium silicate, metal salts of tungstic acid, magnesium, silica, zeolite, bentonite, barium sulfate, calcined calcium sulfate (plaster of Paris), calcium phosphate, fluorapatite, hydroxyapatite, ceramic powder, boron nitride, titanium dioxide, and zinc oxide; organic powders such as polyamide resin powder, nylon powder, polyethylene powder, polypropylene powder, polyester powder, methyl polymethacrylate powder, polystyrene powder, styrene-acrylic acid copolymer resin powder, benzoguanamine resin powder, polytetrafluoroethylene powder, and cellulose powder; and various pigments.

In addition, for example, an antioxidant, a UV absorber, a UV screen, an antiseptic, a moisturizer, a dye, and so on can be formulated therein appropriately. Examples of the UV screen include fine-particle titanium oxide having an average primary particle size of 0.1 µm or smaller and fine-particle zinc oxide having an average primary particle size of 0.1 µm or smaller.

The cosmetic composition of the present invention may be provided in any form and is provided in the form of preferably a solid powder cosmetic composition, particularly preferably, a pressed powder.

The cosmetic composition of the present invention described above in detail is suitable particularly for touch-up. Specifically, the cosmetic composition of the present invention exerts its effect particularly when applied to the made-up skin that has come off over time and become dull.
Accordingly, the present invention provides a cosmetic method performed for touch-up to the made-up skin, comprising applying the cosmetic composition according to the present invention to an area whose makeup has come off.

In the cosmetic method of the present invention, the cosmetic composition of the present invention is applied with a sponge or a cosmetic puff. It is preferable to use a cosmetic puff. The application with the cosmetic puff further reduces a powdery finish, compared with that with a sponge. Particularly, a cosmetic puff as described in, for example, JP-A-2002-262928 or JP-A-2002-262929 is preferably used in which a base material comprising a pile-implanted fabric is disposed on at least one surface (application surface) of a core comprising urethane foam and integrated therewith using an adhesive or suture.

Thus, the present invention also provides a touch-up tool comprising the cosmetic composition of the present invention and a cosmetic puff. The cosmetic composition of the present invention may be contained in an appropriate container. It is preferred that the cosmetic puff should be removably integrated with the container.

### Examples

Hereinafter, the present invention will be described more specifically with reference to Examples. However, the technical scope of the present invention is not restrictively interpreted by them. In the present specification, amounts formulated mean % by mass, unless otherwise specified.
Moreover, evaluation methods and evaluation criteria for sensory tests in Examples below are as follows:

### <Evaluation Method>

Cosmetic compositions (pressed powder) with formulations described in each Example below were prepared, and 10 expert panelists actually used each cosmetic composition. Evaluation items described in each Example were evaluated according to the following evaluation criteria.

### <Evaluation Criteria>

A: very good
B: good
C: ordinary
D: poor

### (Example 1)

Cosmetic compositions (pressed powder) with formulations described in Table 1 below were prepared and evaluated for their removability/applicability, spreadability, absence of powdery finish, and luster finish.

**[Table 1]**

| Formulation | A | B | C |
|---|---|---|---|
| Talc in plate form ¹⁾ | 50 | - | - |
| Talc ²⁾ | - | 50 | - |
| Synthetic mica | 10.84 | 10.84 | 60.84 |
| Pigment-grade titanium oxide | 8 | 8 | 8 |
| Fine-particle titanium oxide | 5 | 5 | 5 |
| Silicone elastomer | 1 | 1 | 1 |
| PMMA | 5 | 5 | 5 |
| Silica | 4 | 4 | 4 |
| Nylon-12 | 3 | 3 | 3 |
| Hydrophobized iron oxide, red | 0.7 | 0.7 | 0.7 |
| Hydrophobized iron oxide, yellow | 2.1 | 2.1 | 2.1 |
| Hydrophobized iron oxide, black | 0.12 | 0.12 | 0.12 |
| Barium sulfate | 3 | 3 | 3 |
| Chlorphenesin | 0.2 | 0.2 | 0.2 |
| Squalane | 2 | 2 | 2 |
| Diphenylsiloxy phenyl trimethicone | 1 | 1 | 1 |
| Octyl methoxycinnamate | 3 | 3 | 3 |
| Sorbitan sesquiisostearate | 1 | 1 | 1 |
| Antioxidant | 0.02 | 0.02 | 0.02 |
| Oxidation inhibitor | 0.02 | 0.02 | 0.02 |
| Total | 100 | 100 | 100 |
| Removability/applicability | B | A | A |
| Spreadability | A | B | B |
| Absence of powdery finish | A | C | D |
| Luster finish | A | D | C |

| | | | |
|---|---|---|---|
| 1) Talc in a plate form having an average particle size (D50) of 20 µm 2) A talc mixture of plate and block forms having an average particle size (D50) of 12.1 µm | | | |

The formulation B that had talc having an average particle size of 12.1 µm instead of the talc in a plate form in the cosmetic composition (formulation A) of the present invention lost a luster finish. The formulation C that had synthetic mica instead thereof particularly had a powdery finish.

### (Example 2)

Cosmetic compositions (pressed powder) with formulations described in Table 2 below were produced and evaluated for their removability/applicability, spreadability, absence of powdery finish, and luster finish.

**[Table 2]**

| Formulation | A | B | C |
|---|---|---|---|
| Talc in plate form ¹⁾ | 50 | - | - |
| Talc in plate form ²⁾ | - | 50 | - |
| Talc in plate form ³⁾ | - | - | 50 |
| Synthetic mica | 10.84 | 10.84 | 10.84 |
| Pigment-grade titanium oxide | 8 | 8 | 8 |
| Fine-particle titanium oxide | 5 | 5 | 5 |
| Silicone elastomer | 1 | 1 | 1 |
| PMMA | 5 | 5 | 5 |
| Silica | 4 | 4 | 4 |
| Nylon-12 | 3 | 3 | 3 |
| Hydrophobized iron oxide, red | 0.7 | 0.7 | 0.7 |
| Hydrophobized iron oxide, yellow | 2.1 | 2.1 | 2.1 |
| Hydrophobized iron oxide, black | 0.12 | 0.12 | 0.12 |
| Barium sulfate | 3 | 3 | 3 |
| Chlorphenesin | 0.2 | 0.2 | 0.2 |
| Squalane | 2 | 2 | 2 |
| Diphenylsiloxy phenyl trimethicone | 1 | 1 | 1 |
| Octyl methoxycinnamate | 3 | 3 | 3 |
| Sorbitan sesquiisostearate | 1 | 1 | 1 |
| Antioxidant | 0.02 | 0.02 | 0.02 |
| Oxidation inhibitor | 0.02 | 0.02 | 0.02 |
| Total | 100 | 100 | 100 |
| Removability/applicability | B | B | A |
| Spreadability | A | B | B |
| Absence of powdery finish | A | B | BC |
| Luster finish | A | B | B |

| | | | |
|---|---|---|---|
| Particle size distribution of talc in a plate form determined by wet-type laser diffraction 1) D50= 18 to 40 um or larger and D10= 8 um or larger 2) D50= 18 to 40 um or larger and D10= 8 um or larger 3) D50= 10 to 25 um or larger and D10= 4 um or larger | | | |

All the formulations A, B, and C described in Table 2 above are Examples of the present invention. However, the formulation A formulated with talc in a plate form from which powder having a small particle size was removed by fractionation treatment was superior particularly in the spreadability and finish of the cosmetic composition to the formulation B formulated with talc in a plate form that had an average particle size equal thereto but underwent no fractionation treatment or the formulation C formulated with talc in a plate form that had an average particle size close to the lower limit and also underwent no fractionation treatment.

### (Example 3)

Cosmetic compositions (pressed powder) with formulations described in Table 3 below were produced and evaluated for their removability/applicability, spreadability, absence of powdery finish, and luster finish.

**[Table 3]**

| Formulation | A | B | C | D | E |
|---|---|---|---|---|---|
| Talc in plate form ¹⁾ | 10 | 30 | 50 | 60 | 70 |
| Talc ²⁾ | 60.04 | 40.04 | 20.04 | 10.04 | 0.04 |
| Synthetic mica | | | | | |
| Pigment-grade titanium oxide | 8 | 8 | 8 | 8 | 8 |
| Silicone elastomer | 1 | 1 | 1 | 1 | 1 |
| PMMA | 3 | 3 | 3 | 3 | 3 |
| Silica | 4 | 4 | 4 | 4 | 4 |
| Nylon-12 | 3 | 3 | 3 | 3 | 3 |
| Hydrophobized iron oxide, red | 0.7 | 0.7 | 0.7 | 0.7 | 0.7 |
| Hydrophobized iron oxide, yellow | 2.1 | 2.1 | 2.1 | 2.1 | 2.1 |
| Hydrophobized iron oxide, black | 0.12 | 0.12 | 0.12 | 0.12 | 0.12 |
| Barium sulfate | 1 | 1 | 1 | 1 | 1 |
| Squalane | 2 | 2 | 2 | 2 | 2 |
| Diphenylsiloxy phenyl trimethicone | 1 | 1 | 1 | 1 | 1 |
| Octyl methoxycinnamate | 3 | 3 | 3 | 3 | 3 |
| Sorbitan sesquiisostearate | 1 | 1 | 1 | 1 | 1 |
| Antioxidant | 0.02 | 0.02 | 0.02 | 0.02 | 0.02 |
| Oxidation inhibitor | 0.02 | 0.02 | 0.02 | 0.02 | 0.02 |
| Total | 100 | 100 | 100 | 100 | 100 |
| Removability/applicability | A | A | A | B | D |
| Spreadability | A | B | B | B | C |
| Absence of powdery finish | C | B | A | A | A |
| Luster finish | C | B | A | A | A |

| | | | | | |
|---|---|---|---|---|---|
| 1) Talc in a plate form having an average particle size (D50) of 20 µm 2) A talc mixture of plate and block forms having an average particle size (D50) of 12.1 µm | | | | | |

The formulation E containing talc in a plate form formulated in an amount exceeding the upper limit of the range specified by the present invention particularly had unsatisfactory removability/applicability of the cosmetic composition. By contrast, the formulations A to D (cosmetic compositions of the present invention) were exceedingly favorable.

### (Example 4)

Cosmetic compositions with formulations described in Table 4 below were prepared and evaluated for their coverage and absence of cakiness.

**[Table 4]**

| Formulation | A | B | C | D | E |
|---|---|---|---|---|---|
| Talc in plate form ¹⁾ | 50 | 50 | 50 | 50 | 50 |
| Synthetic mica | 13.84 | 10.84 | 7.84 | 4.84 | 1.84 |
| Pigment-grade titanium oxide | 3 | 6 | 9 | 12 | 15 |
| Fine-particle titanium oxide | 5 | 5 | 5 | 5 | 5 |
| Silicone elastomer | 3 | 3 | 3 | 3 | 3 |
| PMMA | 5 | 5 | 5 | 5 | 5 |
| Silica | 4 | 4 | 4 | 4 | 4 |
| Nylon-12 | 3 | 3 | 3 | 3 | 3 |
| Hydrophobized iron oxide, red | 0.7 | 0.7 | 0.7 | 0.7 | 0.7 |
| Hydrophobized iron oxide, yellow | 2.1 | 2.1 | 2.1 | 2.1 | 2.1 |
| Hydrophobized iron oxide, black | 0.12 | 0.12 | 0.12 | 0.12 | 0.12 |
| Barium sulfate | 3 | 3 | 3 | 3 | 3 |
| Chlorphenesin | 0.2 | 0.2 | 0.2 | 0.2 | 0.2 |
| Squalane | 2 | 2 | 2 | 2 | 2 |
| Diphenylsiloxy phenyl trimethicone | 1 | 1 | 1 | 1 | 1 |
| Octyl methoxycinnamate | 3 | 3 | 3 | 3 | 3 |
| Sorbitan sesquiisostearate | 1 | 1 | 1 | 1 | 1 |
| Antioxidant | 0.02 | 0.02 | 0.02 | 0.02 | 0.02 |
| Oxidation inhibitor | 0.02 | 0.02 | 0.02 | 0.02 | 0.02 |
| Total | 100 | 100 | 100 | 100 | 100 |
| Coverage | D | C | B | A | A |
| Absence of cakiness | A | B | B | C | D |

| | | | | | |
|---|---|---|---|---|---|
| 1) Talc in a plate form having an average particle size (D50) of 20 µm | | | | | |

The formulation A containing pigment-grade titanium oxide only in an amount smaller than the range specified by the present invention particularly had an insufficient coverage. The formulation E formulated with a large amount of pigment-grade titanium oxide tended to have a sufficient coverage but a cakey finish.

### (Example 5)

Cosmetic compositions (pressed powder) with formulations described in Table 5 below were produced and evaluated for its removability/applicability, spreadability, fit, and impact resistance.

**[Table 5]**

| Formulation | A | B | C | D | E |
|---|---|---|---|---|---|
| Talc in plate form ¹⁾ | 50 | 50 | 50 | 50 | 50 |
| Synthetic mica | 22.08 | 21.08 | 19.08 | 17.08 | 15.08 |
| Pigment-grade titanium oxide | 8 | 8 | 8 | 8 | 8 |
| Silicone elastomer | 1 | 1 | 1 | 1 | 1 |
| PMMA | 3 | 3 | 3 | 3 | 3 |
| Silica | 4 | 4 | 4 | 4 | 4 |
| Nylon-12 | 3 | 3 | 3 | 3 | 3 |
| Hydrophobized iron oxide, red | 0.7 | 0.7 | 0.7 | 0.7 | 0.7 |
| Hydrophobized iron oxide, yellow | 2.1 | 2.1 | 2.1 | 2.1 | 2.1 |
| Hydrophobized iron oxide, black | 0.12 | 0.12 | 0.12 | 0.12 | 0.12 |
| Barium sulfate | 1 | 1 | 1 | 1 | 1 |
| Squalane | 1.43 | 1.716 | 2.288 | 2.86 | 3.432 |
| Diphenylsiloxy phenyl trimethicone | 0.72 | 0.864 | 1.152 | 1.44 | 1.728 |
| Octyl methoxycinnamate | 2.14 | 2.568 | 3.424 | 4.28 | 5.136 |
| Sorbitan sesquiisostearate | 0.71 | 0.852 | 1.136 | 1.42 | 1.704 |
| Total | 100 | 100 | 100 | 100 | 100 |
| Amount of oil | 5 | 6 | 8 | 10 | 12 |
| Removability/applicability | A | A | B | B | D |
| Spreadability | A | A | B | C | D |
| Fit | D | C | B | A | A |
| Impact resistance | D | B | B | A | A |

| | | | | | |
|---|---|---|---|---|---|
| 1) Talc in a plate form having an average particle size (D50) of 20 µm | | | | | |

The formulation A containing oil only in an amount smaller than the range specified by the present invention particularly had an insufficient fit and impact resistance. The formulation E formulated with a large amount of oil had a sufficient fit and impact resistance but was inferior particularly in the removability/applicability and spreadability of the cosmetic composition.

### (Example 6)

Cosmetic compositions (pressed powder) with formulations described in Table 6 below were produced and evaluated for its removability/applicability, spreadability, absence of powdery finish, and luster finish.

**[Table 6]**

| Formulation | A | B | C |
|---|---|---|---|
| Talc in plate form ¹⁾ | 50 | 50 | 50 |
| Synthetic mica | 10.84 | 10.84 | 10.84 |
| Pigment-grade titanium oxide | 8 | 8 | 8 |
| Fine-particle titanium oxide | 5 | 5 | 5 |
| Silicone elastomer | 1 | 1 | 1 |
| PMMA | 5 | 5 | 5 |
| Silica | 4 | 4 | 4 |
| Nylon-12 | 3 | 3 | 3 |
| Hydrophobized iron oxide, red | 0.7 | 0.7 | 0.7 |
| Hydrophobized iron oxide, yellow | 2.1 | 2.1 | 2.1 |
| Hydrophobized iron oxide, black | 0.12 | 0.12 | 0.12 |
| Barium sulfate | 3 | 3 | 3 |
| Chlorphenesin | 0.2 | 0.2 | 0.2 |
| Squalane | 2 | | |
| Diphenylsiloxy phenyl trimethicone | 1 | | |
| Methylpolysiloxane 1000 cs | | 3 | |
| Methylpolysiloxane 5000 cs | | | 3 |
| Octyl methoxycinnamate | 3 | 3 | 3 |
| Sorbitan sesquiisostearate | 1 | 1 | 1 |
| Antioxidant | 0.02 | 0.02 | 0.02 |
| Oxidation inhibitor | 0.02 | 0.02 | 0.02 |
| Total | 100 | 100 | 100 |
| Removability/applicability | B | C | D |
| Spreadability | A | C | D |
| Absence of powdery finish | A | B | C |
| Luster finish | A | C | C |

| | | | |
|---|---|---|---|
| 1) Talc in a plate form having an average particle size (D50) of 20 µm Viscosity (25°C) of oil mixture: Formulation A: 25 cps Formulation B: 100 cps Formulation C: 2000 cps | | | |

The formulation C containing oil having a viscosity exceeding 100 cps had poor removability/applicability and spreadability of the cosmetic composition and an insufficient touch of finish.

### (Example 7)

Cosmetic compositions (pressed powder) with formulations described in Table 7 below were produced and evaluated for its removability/applicability, spreadability, absence of powdery finish, and impact resistance.

**[Table 7]**

| Formulation | A | B | C | D |
|---|---|---|---|---|
| Talc in plate form ¹⁾ | 50 | 50 | 50 | 50 |
| Synthetic mica | 11.84 | 8.84 | 6.84 | 4.84 |
| Pigment-grade titanium oxide | 8 | 8 | 8 | 8 |
| Fine-particle titanium oxide | 5 | 5 | 5 | 5 |
| Silicone elastomer powder | 0 | 3 | 5 | 7 |
| PMMA | 5 | 5 | 5 | 5 |
| Silica | 4 | 4 | 4 | 4 |
| Nylon-12 | 3 | 3 | 3 | 3 |
| Hydrophobized iron oxide, red | 0.7 | 0.7 | 0.7 | 0.7 |
| Hydrophobized iron oxide, yellow | 2.1 | 2.1 | 2.1 | 2.1 |
| Hydrophobized iron oxide, black | 0.12 | 0.12 | 0.12 | 0.12 |
| Barium sulfate | 3 | 3 | 3 | 3 |
| Chlorphenesin | 0.2 | 0.2 | 0.2 | 0.2 |
| Squalane | 2 | 2 | 2 | 2 |
| Diphenylsiloxy phenyl trimethicone | 1 | 1 | 1 | 1 |
| Octyl methoxycinnamate | 3 | 3 | 3 | 3 |
| Sorbitan sesquiisostearate | 1 | 1 | 1 | 1 |
| Antioxidant | 0.02 | 0.02 | 0.02 | 0.02 |
| Oxidation inhibitor | 0.02 | 0.02 | 0.02 | 0.02 |
| Total | 100 | 100 | 100 | 100 |
| Removability/applicability | C | B | A | A |
| Spreadability | C | B | B | B |
| Absence of powdery finish | B | A | B | C |
| Impact resistance | B | B | C | D |

| | | | | |
|---|---|---|---|---|
| 1) Talc in a plate form having an average particle size (D50) of 20 µm | | | | |

The formulations B and C formulated with silicone elastomer powder were superior in usability such as the removability/applicability of the cosmetic composition and the absence of a powdery finish to the formulation A free from silicone elastomer powder. However, the formulation D containing silicone elastomer powder formulated in an amount of 7% by mass was excellent in usability such as the removability/applicability but had insufficient impact resistance.

## Claims

1. A solid powdery cosmetic composition for touch-up comprising the following a) to c) :
a) 10 to 60% of talc in a plate form having an average particle size D50 of 15 to 40 µm;
b) 6 to 12% of pigment-grade titanium oxide; and
c) 6 to 9% of oil.

2. The solid powdery cosmetic composition according to claim 1, wherein the talc in a plate form has an average particle size D10 of 4 µm or larger.

3. The solid powdery cosmetic composition according to claim 1 or 2, wherein the talc in a plate form has an average aspect ratio of 10 to 100.

4. The solid powdery cosmetic composition according to any one of claims 1 to 3, wherein the oil has a viscosity of 100 cps or lower.

5. A touch-up tool comprising a cosmetic composition according to any one of claims 1 to 4 and a cosmetic puff.

6. A cosmetic method performed for touch-up to the made-up skin, comprising applying a solid powdery cosmetic composition according to any one of claims 1 to 4 to an area whose makeup has come off.

7. The cosmetic method according to claim 6, wherein the application is performed using a cosmetic puff.
